# EUROPÄISCHE PATENTSCHRIFT

(11) **EP 2 428 558 B1**
(45) Veröffentlichungstag und Bekanntmachung des Hinweises auf die Patenterteilung: **23.10.2013**
(21) Anmeldenummer: 10009480.4
(22) Anmeldetag: 13.09.2010
(51) Int. Cl.: C12M 1/107

(54) **Fermenter zur Vergärung von Biomasse**
Fermenter for fermenting biomass
Fermenteur destiné à la fermentation de biomasse

(43) Veröffentlichungstag der Anmeldung: 14.03.2012
(73) Patentinhaber: KOMPOFERM GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- EP-A1- 1 428 868
- EP-A1- 1 736 535
- EP-A1- 2 062 965
- EP-A2- 0 350 455
- WO-A2-2005/054423
- DE-A1- 10 115 623
- DE-A1-102007 025 807
- DE-U1-202006 014 149

## Beschreibung

Die Erfindung betrifft einen Fermenter zur Vergärung von Biomasse nach dem Oberbegriff des Anspruchs 1.

Derartige Fermenter sind aus dem Stand der Technik Bekannt. Sie dienen der Fermentierung fester oder halbfester Biomasse, welche in Form einer Schüttung oder eines Haufwerks in den Fermenter eingebracht wird. Konventionelle Fermenter sind dabei häufig als garagenförmige Bauwerke ausgebildet, so dass die Biomasse in vergleichsweise einfacher Weise, beispielsweise mit Radladern, in den Fermenter eingebracht werden kann.

Abhängig von dem jeweiligen Fermentationsprozess weisen die Fermenter in ihrem Inneren häufig zusätzliche Einbauten, beispielsweise Berieselungsanlagen an der Fermenterdecke oder Belüftungseinrichtungen auf, um den Fermentationsprozess beeinflussen zu können. Aufgrund der bei der Fermentation entstehenden Gase ist dabei eine gasdichte Bauweise des Fermenters von Bedeutung, ebenfalls erfordern die Fermenter abhängig vom Klima des Aufstellungsortes mehr oder weniger aufwändige Isolationsschichten, um eine für die Fermentation geeignete Prozesstemperatur im Fermenter aufrecht zu erhalten.

WO 2005/054423 offenbart einen Fermenter nach dem Oberbegriff des Anspruchs 1. Dieser verfügt über ein nicht näher dargestelltes Drainagesystem. Das kennzeichnende Merkmal, dass sich dies auf der von der eingebrachten Biomasse weg weisenden Seite einer Stützvorrichtung befindet, wird jedoch in der D1 nicht offenbart.

Weiter ist auch aus DE 20 2006 014149 U1 ein gattungsgemäßer Fermenter mit Drainagesystem bekannt. Die D2 offenbart einen Behälter für die Flüssigfermentation, bei dem die Mantelfläche durch eine Konstruktion aus einer Membran und einem stabilisierenden Gitternetz gebildet ist.

Weiterhin ist aus der EP 1 428 868 A1 ein Garagenfermenter mit einem gasdurchlässigen Belüftungsboden bekannt. Bei dem Boden dieses Fermenters handelt es sich um einen Boden, der zwar die Gaszufuhr und Perkolatabfuhr erlaubt, jedoch für einen mobilen bzw. semi-mobilen Einsatz des Fermenters nicht geeignet ist. Der Boden weist eine Doppelbodenkonstruktion auf, bei der die Böden einen sich flächig unter dem Fermenter erstreckenden Freiraum einschließen. Auf diese Weise wird zwar die Gaszufuhr und Perkolatabfuhr ermöglicht, gleichzeitig jedoch die Struktur im Bodenbereich des Fermenters geschwächt, so dass für einen semi-mobilen Einsatz bei einer derartigen Konstruktion entweder ein zu hohes Gewicht des Fermenters entsteht oder die Stabilität des Fermenters für die notwendigen Transportvorgänge nicht ausreicht.

Der Aufbau derartiger garagenförmiger Gebäude muss aufgrund deren Größe, welche notwendig ist, um einen wirtschaftlichen Fermentationsbetrieb zu ermöglichen, an deren Aufstellungsort erfolgen. Dabei kommen überwiegend konventionelle Bautechniken zum Einsatz, was mit den entsprechenden Kosten und logistischen Nachteilen, die sich vor allem aus der vergleichsweise hohen Bauzeit ergeben, verbunden ist. Dementsprechend sind derartige Anlagen oft schwierig erweiterbar, eine verkleinerung einer bestehenden Anlage ist zudem in der Regel nicht ohne den Verlust der Kosten für die getätigten Investitionen möglich.

Der Erfindung liegt daher die Aufgabe zugrunde, einen Fermenter zu schaffen, der den kostengünstigen Aufbau insbesondere kleiner und mittlerer Fermentationsanlagen sowie vorzugsweise die Möglichkeit einer einfachen Erweiter- und/ oder Verkleinerbarkeit der Fermentationsanlage bei zugleich möglichst günstigen Fermentationsbedingungen im Inneren des Fermenters schafft.

Die Aufgabe wird gelöst durch einen Fermenter mit den Merkmalen des Anspruchs 1. Vorteilhafte Ausführungsformen finden sich in den Unteransprüchen.

Erfindungsgemäß wird die Aufgabe dadurch gelöst, dass der Fermenter durch das Zusammenwirken wenigstens der Sohle mit einer die Fermenterkonstruktion nicht tragenden Hülle gasdicht verschlossen ist. Dabei ist die Hülle so angebracht, dass die in dem Fermenter eingebrachte Biomasse sich lediglich auf der Sohle des Fermenters und/oder einer Stützvorrichtung abstützt.

Erfindungsgemäß ist damit eine Trennung der Funktionen des gasdichten Verschließens durch die die Fermenterkonstruktion nicht tragende Hülle der zu fermentierenden Biomasse gegenüber der Umgebung von der Funktion des Tragens und Abstützens der als Haufwerk oder Schüttgut eingebrachten Biomasse durch die Stützvorrichtung getrennt. Dies geht mit einer Gewichtsersparnis einher, die es ermöglicht, eine semi-mobile Fermenterkonstruktion zu schaffen. Der erfindungsgemäße Fermenter kann auf diese Weise industriell vorgefertigt werden, während am Aufstellungsort lediglich die Aufstellungsflächen vorbereitet, d.h. gegebenenfalls Fundamente und/oder Unterkellerungen vorbereitet werden müssen. Nach Abschluss der vorbereitungsarbeiten können dann die bereits vorgefertigten Fermenter in kurzer Zeit aufgestellt werden, wobei es mit vertretbarem Aufwand möglich ist, weitere Fermenter hinzuzufügen oder zu entfernen, wobei durch die Abtransportierbarkeit einzelner Fermenter deren Wiederveräußerung möglich ist. Alternativ ist es auch denkbar, dass lediglich die vorbereiteten Aufstellungsflächen vom Betreiber gestellt werden, während der Hersteller der Fermenter diese nach Art eines Leasingkonzeptes zur Verfügung stellt. Das Zusammenwirken wenigstens der Sohle mit der die Fermenterkonstruktion nicht tragenden Hülle bedeutet in diesem Zusammenhang, dass diese beiden Elemente einen wesentlichen Teil der gesamten gasdichten Hülle des Fermenters ausmachen. Dies bedeutet, dass durchaus weitere Elemente, beispielsweise ein gasdichtes Tor oder eine Wand bzw. ein Teil einer Wand vorgesehen sein können, die ebenfalls Teile der gesamten gasdichten Hülle des Fermenters ausmachen, d.h. Sohle und die die Fermenterkonstruktion nicht tragenden Hülle sind nicht zwingend die einzigen Elemente, die alleine den gasdichten Aufbau des Fermenters sicherstellen.

Die Sohle des erfindungsgemäßen Fermenters weist dabei ein auf der von der eingebrachten Biomasse weg weisenden Seite der Stützvorrichtung angeordnetes Drainagesystem auf. Das Drainagesystem dient der Abfuhr von Flüssigkeiten, die aus der Biomasse austreten, insbesondere der Ab-/Rückfuhr von Perkolat. Die erfindungsgemäße Anordnung in Bezug auf die Stützvorrichtung bringt den Vorteil mit sich, dass die Sohle des Fermenters durch das eingelassene Drainagesystem nicht in dem Bereich geschwächt wird, in dem die Biomasse aufgenommen wird. Dies ist insbesondere deswegen von Bedeutung, da das Eigengewicht der Sohle für die angestrebte semi-mobile Verwendbarkeit möglichst niedrig sein muss, da die Sohle im beladbaren Bereich des Fermenters nicht nur das Gewicht der Biomasse, sondern auch das zum Beladen des Fermenters eingesetzter Hilfsmittel, wie beispielsweise Radlader, aufnehmen muss, was zu einer punktuell um ein Vielfaches erhöhten Belastung führt. Weiterhin ermöglicht die erfindungsgemäße Anordnung eine besonders günstige Perkolatstromführung, wenn dieses seitlich aus dem Haufwerk durch die Stützvorrichtung austreten kann. Insbesondere wenn der Boden ein Belüftungssystem für das Haufwerk aufweist, kommen die mechanischen und prozesstechnischen Vorteile (Perkolatstromführung) zum Tragen.

Eine bevorzugte Ausführungsform sieht eine starre Tragstruktur zur Abstützung und/oder Aufhängung der die Fermenterkonstruktion nicht tragenden Hülle vor.

Eine derartig starre Tragstruktur muss nicht als geschlossene Hülle ausgeführt sein, was den Bau einer vergleichsweise leichten Tragstruktur ermöglicht, welche die Mobilität der Fermenter unwesentlich einschränkt, jedoch die Möglichkeit eröffnet, zusätzliche Module der Fermenteranlage auf die Fermenter aufzustapeln. Dabei kann es sich beispielsweise um Pumpen und/oder Verdichter für Gas- bzw. Flüssigkeitskreisläufe, die für die Fermentation benötigt werden, handeln. Ebenso kann eine beispielsweise in einer Art Container integrierte Steuerwarte auf den Fermentern aufgestapelt werden. Auf diese Weise wird keine über die Grundfläche der aufzustellenden Fermenter hinausgehende Flächenvorbereitung erforderlich und es ergibt sich die Möglichkeit, die gesamte Anlage, d.h. nicht nur die Fermenter selbst, sondern auch die notwendige technische Peripherie, modular zu erweitern, zu verkleinern oder zur Verfügung zu stellen.

Vorteilhafterweise ist die starre Tragstruktur dabei nach Art eines Gitters ausgebildet. Eine derartige Gitterkonstruktion vereint ein niedriges Eigengewicht mit einer hohen Tragfähigkeit und ist darüber hinaus kostengünstig herzustellen.

Vorzugsweise ist die die Fermenterkonstruktion nicht tragende Hülle nach Art eines Sacks ausgebildet. Das sackartige Gebilde erstreckt sich dabei vorteilhafterweise von einem an der Stirnseite des Fermenters vorzusehenden Tor zur Beladung des Fermenters mit Biomasse über die Länge der Grundfläche der Sohle des Fermenters. Dabei stellt die das Tor aufweisende Stirnseite eine der kleinflächigen Stirnseiten des Fermenters dar, zu der sich die Haupterstreckungsrichtung des Fermenters vorzugsweise rechtwinklig erstreckt.

Vorzugsweise weist die die Fermenterkonstruktion nicht tragende Hülle dabei eine Kunststofffolie als wesentlichen Bestandteil auf. Wesentlicher Bestandteil bedeutet in diesem Zusammenhang, dass die Kunststofffolie der Hauptbestandteil in der Hülle ist, und zwar im Hinblick auf seine Funktion der Gasabdichtung. Natürlich kann eine derartige Hülle weitere Bestandteile, beispielsweise Beschichtungen, die beispielsweise dem UV- oder Witterungsschutz dienen können, aufweisen und ebenso Gewebeschichten, die beispielsweise dem mechanischen Schutz der Hülle dienen, beispielsweise um Beschädigungen zu verhindern, wenn beispielsweise beim Beladen die Biomasse in Kontakt mit der Hülle kommt.

Vorzugsweise ist die die Fermenterkonstruktion nicht tragende Hülle dabei innerhalb der starren Tragstruktur angeordnet. Dies hat den Vorteil, dass bei einer Nutzung der Tragstruktur von deren Außenseite, beispielsweise beim Aufstapeln weiterer Module, eine Beschädigung der nicht tragenden Hülle weitestgehend ausgeschlossen ist.

Vorzugsweise ist die die Fermenterkonstruktion nicht tragende Hülle flexibel und an der starren Tragstruktur aufgehängt.

Eine flexible Hüllengestaltung ergibt sich in der Regel bei der gewichtsoptimierten Ausführung einer derartigen Hülle, deren wesentliche Funktion die Gasabdichtung ist, automatisch. Aufgrund der im Fermenterbetrieb entstehenden Gase ist diese Hülle selbsttragend, da leicht an der Innenseite ein entsprechender Überdruck erzeugt werden kann. Kritisch ist lediglich der Moment der Be- und Entladung des Fermenters mit Biomasse, da hierfür der Fermenter geöffnet werden muss und eine flexible Hülle in sich zusammensacken kann. Daher ist es vorteilhaft, diese an der starren Tragstruktur aufzuhängen, so dass die Hülle auch bei einem nicht vorhandenen Überdruck im Fermenter nicht in sich zusammensackt und so das Be- und/oder Entladen des Fermenters mit Biomasse behindert. Neben der Behinderung des Be- und/oder Entladevorgangs trägt diese Maßnahme auch zum Schutz der Hülle vor mechanischen Beschädigungen bei, da diese durch die Aufhängung an der Decke vor dem Arbeitsbereich beispielsweise zum Be- und/oder Entladen genutzter Radlader weggehalten wird. Besonders bervorzugt ist die Aufhängung der Fermenterhülle über eine Berieselungseinrichtung realisiert. Die Berieselungseinrichtung, die auch unabhängig von der Aufhängung der Fermenterhülle vorgesehen sein kann, dient zur Verteilung von Flüssigkeiten, insbesondere Perkolat, über dem Biomassehaufwerk. Vorzugsweise kann die Berieselungseinrichtung in ihrer Höhe verstellt werden, beispielsweise um den Be- und/oder Entladevorgang nicht zu behindern. Die Berieselungseinrichtung kann dabei über Leitungen versorgt werden, die beispielsweise durch eine Rückwand des Fermenters geführt und beispielsweise an der starren Tragstruktur gehalten sind.

Vorzugsweise ist die Sohle derart gestaltet, dass sie eine Bodenplatte aus Beton oder verstärktem Beton, insbesondere aus Stahlbeton aufweist. Die Ausführung der Sohle aus Beton ermöglicht es auf kostengünstige Weise, Installationen, beispielsweise die Verankerung der Tragstruktur, der Stützvorrichtung und/oder der Installationsleitungen zur Versorgung des Fermenterinneren bei der Herstellung des Fermenters in die Sohle einzubringen. Dabei können Anschlüsse für die Zu- und/oder Abfuhr der Medien des Fermenters derart gestaltet werden, dass durch standardisierte Platzierung der Anschlüsse nebeneinander aufgestellte Fermenter in einfacher Weise miteinander verschaltet werden können, wobei es möglich ist, Versorgungsleitungen durch die Behälter, vorzugsweise geradlinig von einer auf die andere Behälterseite durchzuschleifen und eine nebeneinander aufgestellte und mit ihren Versorgungsleitungen miteinander verbundene Anordnung von Behältern an eine zentrale Versorgung anzuschließen, was eine vorteilhafte Parallelschaltung der Medienversorgung der einzelnen Fermenter ermöglicht.

Erfindungsgemäß weist die Sohle dabei ein Belüftungssystem auf. Ein Belüftungssystem, welches Versorgungsleitungen aufweist, die vorzugsweise in der Sohle des Fermenters verlegt sind, sowie daran anschließende Luft- bzw. Gasaustrittsdüsen, welche vorzugsweise über die Fläche der Sohle verteilt sind, bringt den Vorteil mit sich, dass für die Fermentation benötigte Medien, insbesondere Luft, von unten in die Biomasse eingebracht werden können.

Vorteilhafterweise ist die die Fermenterkonstruktion nicht tragende Hülle in einem unteren Bereich zumindest einer Seitenwand als starre Wand ausgeführt. Die starr-wandige Konstruktion der Seitenwand im unteren Bereich als starre Wand, beispielsweise aus einem Stahlblech, hat den Vorteil, dass dieses in einfacher Weise permanent mit der Sohle gasdicht verbunden, insbesondere in diese eingegossen werden kann, insbesondere wenn es sich um eine Betonsohle handelt, und die Verbindung eines flexiblen Teils der nicht tragenden Hülle mit dem als starre Wand ausgeführten Teil der die Fermenterkonstruktion nicht tragenden Hülle damit in einem leicht zugänglichen Bereich liegt, was insbesondere von Vorteil ist, wenn der flexible Teil der Hülle, welcher naturgemäß anfälliger für Beschädigungen ist, einzeln ausgetauscht werden muss. Des Weiteren hat die starre Ausführung den Vorteil, dass sich unter Umständen im Fermenter ansammelnde Flüssigkeiten von einer starren Wand besser nach Art eines Wehrs zurückgehalten werden können als durch eine flexible Hülle.

Vorteilhafterweise sind eine Mehrzahl Fermenter in einer weiteren Umhüllung angeordnet, wobei die weitere Umhüllung einen niedrigeren Wärmedurchgangskoeffizienten aufweist als die die Fermenterkonstruktion nicht tragende Hülle. Die Möglichkeit, modulare Fermenteranlagen mit den erfindungsgemäßen Fermentern aufzubauen, ermöglicht zudem eine weitere Optimierung der Fermenterhülle im Hinblick auf die Trennung der Funktionen Gasdichtkeit und Wärmeisolation. So können bei den erfindungsgemäßen Fermentern mehrere Fermenter nebeneinander aufgestellt und dann mit einer gemeinsamen weiteren Umhüllung, die lediglich die Funktion der Wärmeisolation erfüllen muss, versehen werden. So können beispielsweise in einfachster Weise Dämmplatten, beispielsweise aus Polystyren oder Mineralwolle um die aufgebauten Fermenter angeordnet werden. Vorteilhafterweise kann die Befestigung dabei in einfacher Weise an der starren Tragstruktur der Fermenter erfolgen.

Vorteilhafterweise weist die Sohle Durchlässe für ein flüssiges Medium auf. Dies hat den Vorteil, dass ein flüssiges Medium, beispielsweise eine mit Mikroorganismen beladene Flüssigkeit, sogenanntes Perkolat, durch die Sohle in und/ oder aus dem Behälter geführt werden kann. Das Vorsehen der Perkolatanschlüsse in der Sohle des Behälters ist insbesondere dann vorteilhaft, wenn der Behälter sich oberhalb einer Unterkellerung befindet, welche als Speicher für ein flüssiges Medium genutzt wird. Im Falle des Perkolats wird eine derartige Unterkellerung auch als Perkolatspeicher bezeichnet.

Vorteilhafterweise ist eine Leitung, insbesondere eine Steigleitung für ein flüssiges Medium vorgesehen, die sich vorzugsweise in Höhenrichtung des Fermenters mindestens vom Bereich der Sohle bis zur für die eingebrachte Biomasse vorgesehenen Haufwerkshöhe erstreckt. Die Leitung befindet sich dabei vorzugsweise innerhalb der die Fermenterkonstruktion nicht tragenden Hülle und hat den Vorteil, dass ein flüssiges Medium, beispielsweise Perkolat, mit dieser Leitung auf ein Höhenniveau oberhalb des Haufwerkscheitelpunkts gebracht werden kann, das es ermöglicht, dann das flüssige Medium oberhalb des Haufwerks zu verteilen und, beispielsweise durch geeignete Düsen, derart auf das Haufwerk aufzubringen, dass es durch dieses aufgrund seiner Schwerkraft hindurch rieseln kann.

Die Erfindung wird nachstehend anhand der Zeichnungen im Einzelnen näher erläutert:
- Figur 1 -: zeigt eine Schnittdarstellung eines beispielhaften erfindungsgemäßen Fermenters,
- Figur 2 -: zeigt eine Schnittdarstellung eines beispielhaften erfindungsgemäßen Fermenters in einer weiteren Ausführungsform,
- Figur 3 -: zeigt eine Anordnung beispielhafter erfindungsgemäßer Fermenter in einer Grundrissdarstellung,
- Figur 4 -: zeigt einen Längsschnitt durch einen beispielhaften erfindungsgemäßen Fermenter,

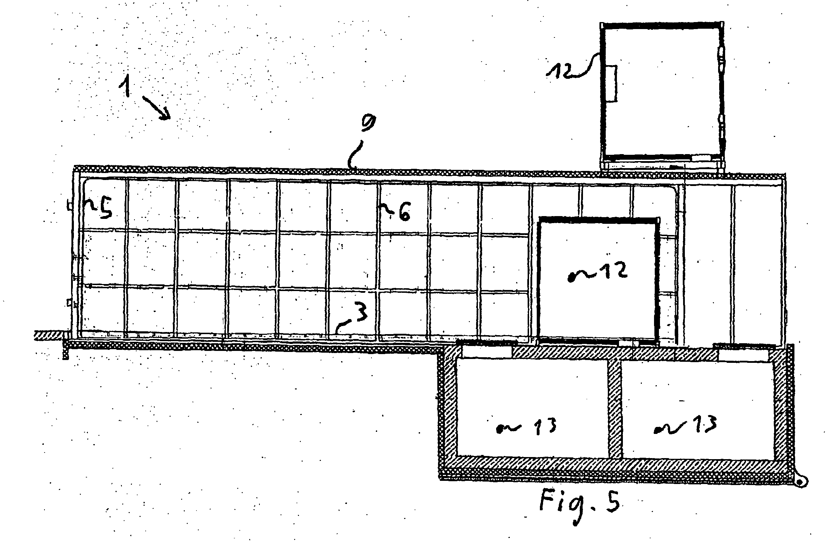

- Figur 5 -: zeigt einen Längsschnitt durch einen teilunterkellerten beispielhaften erfindungsgemäßen Fermenter,

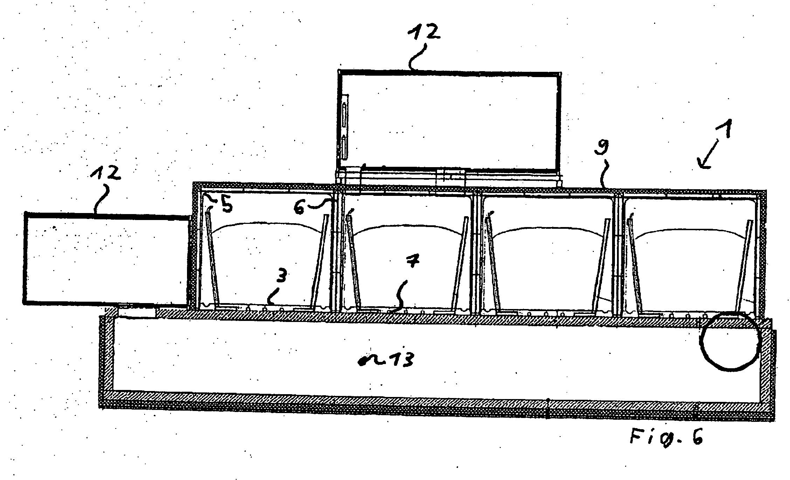

- Figur 6 -: zeigt eine Schnittdarstellung einer Fermenteranlage mit beispielhaften erfindungsgemäßen Fermentern,

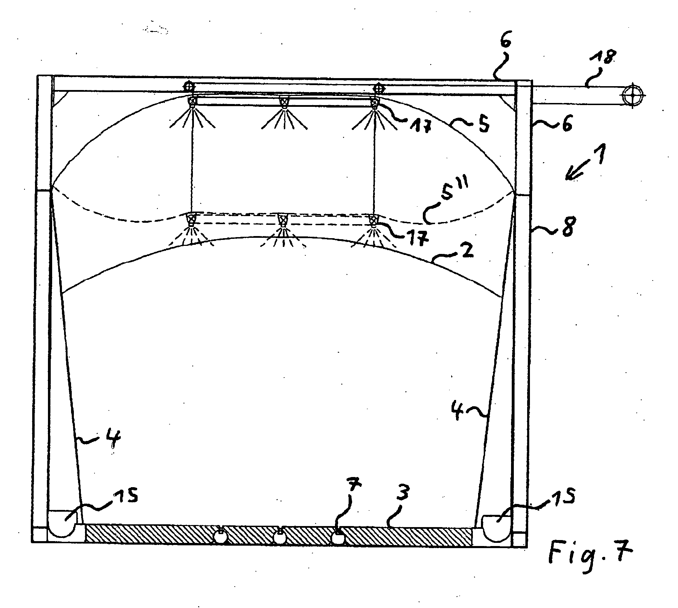

- Figur 7 -: zeigt eine Schnittdarstellung eines beispielhaften erfindungsgemäßen Fermenters mit einer höhenverstellbaren Berieselungseinrichtung,

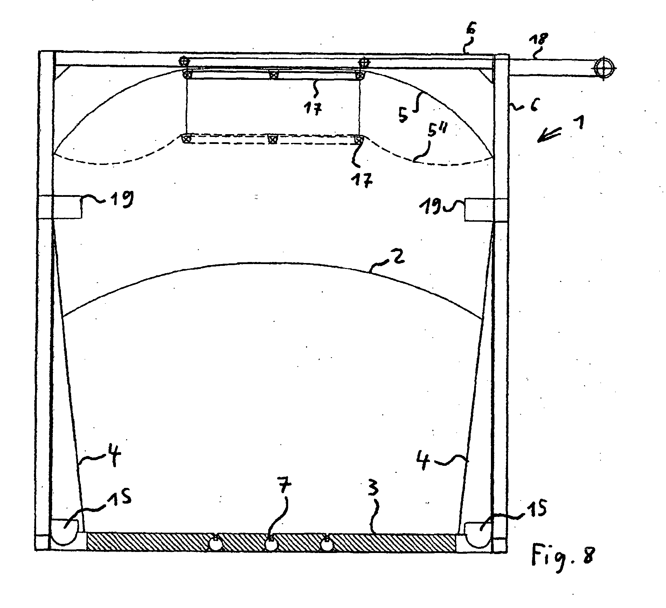

- Figur 8 -: zeigt eine Schnittdarstellung eines beispielhaften erfindungsgemäßen Fermenters mit einer höhenverstellbaren Berieselungseinrichtung.

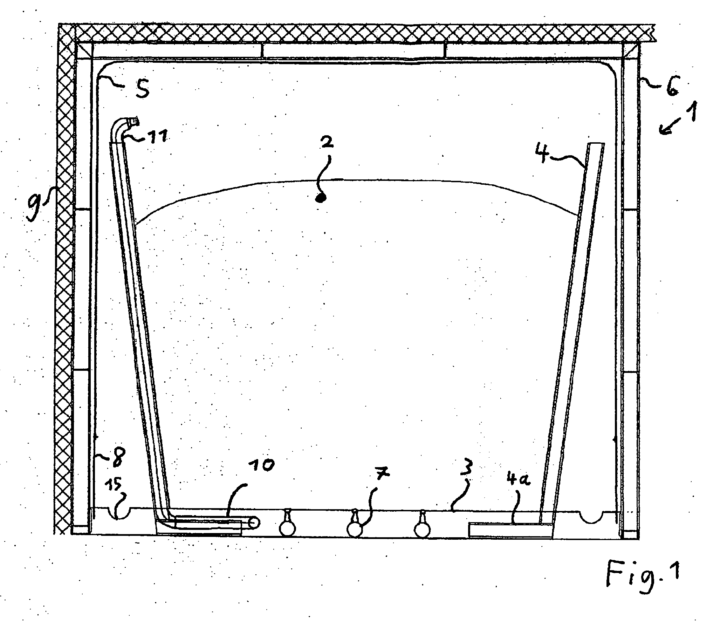

Der in Figur 1 dargestellte beispielhafte Fermenter 1 weist eine Sohle 3 und eine Stützvorrichtung 4, die beispielsweise aus Lochblechen aufgebaut sein kann, auf, welche die Abstützung bzw. Aufnahme der Biomasse 2 übernehmen. Der gasdichte Verschluss des Fermenters wird gewährleistet durch die die Fermenterkonstruktion nicht tragende Hülle 5, die im oberen Bereich des Fermenters flexibel ausgebildet ist. Im unteren Bereich des Fermenters ist die Hülle 5 als starre Wand 8 ausgeführt, die wiederum in der Sohle 3 des Fermenters verankert ist. Die Sohle 3 weist als Rinnen ausgeführte Kanäle 15 auf, die der Abfuhr flüssiger Medien aus dem Fermenter dienen. Weiterhin weist die Sohle 3 ein Belüftungssystem 7 auf, welches beispielhaft so ausgeführt ist, dass die Biomasse 2 durch flächig über den Fermenterboden verteilte Düsen von unten her mit einem Gas, beispielsweise mit Luft, beaufschlagt werden kann.

Die beispielhafte Stützvorrichtung 4 ist im gezeigten Beispiel in der Fermentersohle 3 verankert. Im gezeigten Beispiel kann dies dadurch geschehen, dass Teilbereiche 4a der Stützvorrichtung 4 in die Sohle 3, die beispielsweise aus Beton besteht, eingegossen werden.

Weiterhin weist der beispielhafte Fermenter 1 eine Steigleitung 11 auf, mit der ein flüssiges Medium von Durchlässen 10 in der Sohle 3 des Fermenters auf ein Höhenniveau oberhalb der Haufwerkshöhe der Biomasse 2 gebracht werden kann, wodurch beispielsweise die Berieselung der Biomasse 2 mit Perkolat ermöglicht wird.

Die Hülle 5 ist innerhalb einer starren Tragstruktur 6 angeordnet, an der eine weitere, der Wärmeisolation dienende Umhüllung 9 aufgenommen ist. Dabei ist die Umhüllung 9 so ausgeführt, dass sie sich über mehrere Fermenter erstrecken kann.

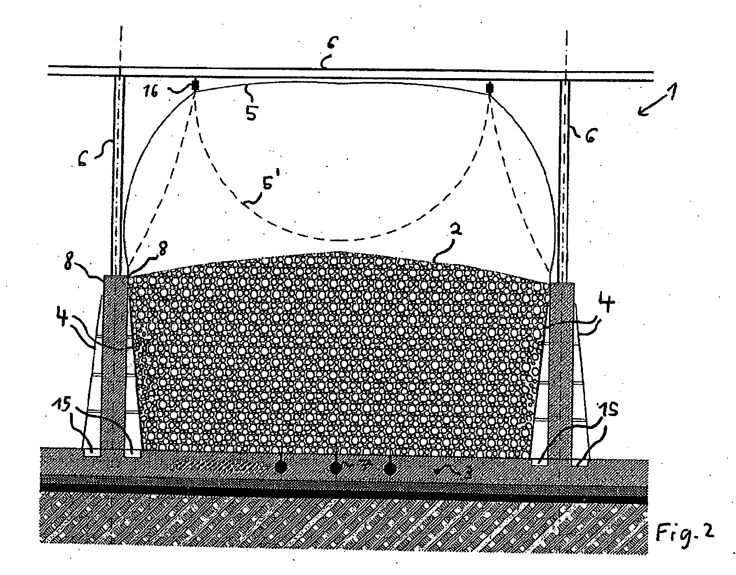

Figur 2 zeigt eine alternative Ausführung des Fermenters 1. Dabei ist eine starre Wand 8 vorgesehen, die im unteren Bereich des Fermenters für den gasdichten Abschluss zwischen der flexiblen Hülle 5 und der Sohle 3 des Fermenters sorgt. An der starren Wand 8 ist im gezeigten Beispiel die Stützvorrichtung 4 aufgenommen, die die Biomasse 2 seitlich abstützt. Die flexible Hülle 5 ist über die Aufhängepunkte 16 an der starren Tragstruktur 6 befestigt. Durch diese Aufhängepunkte wird die Hülle 5, wenn sie beispielsweise beim Be- und/oder Entladen des Fermenters 1 mit Biomasse 2 nicht durch einen Überdruck im Inneren des Fermenters aufgebläht wird, in ihrer Position 5' gehalten, um ein Be- und/oder Entladen des Fermenters 1 mit der Biomasse 2 zu ermöglichen. Vorzugsweise ist die Aufhängung an den Aufhängepunkten 16 so gestaltet, dass die die Fermanterkonstruktion nicht tragende Hülle 5 absenkbar ist (abgesenkte Position 5"). Dies kann bevorzugt durch die Aufnahme der die Fermenterkonstruktion nicht tragenden Hülle 5 an einer, vorzugsweise höhenverstellbaren Berieselungseinrichtung 17 erfolgen, die durch in den Fermenter führende Versorgungsleitungen 18 mit dem über der Biomasse 2 zu verteilenden Medium, insbesondere Perkolat, versorgt wird.

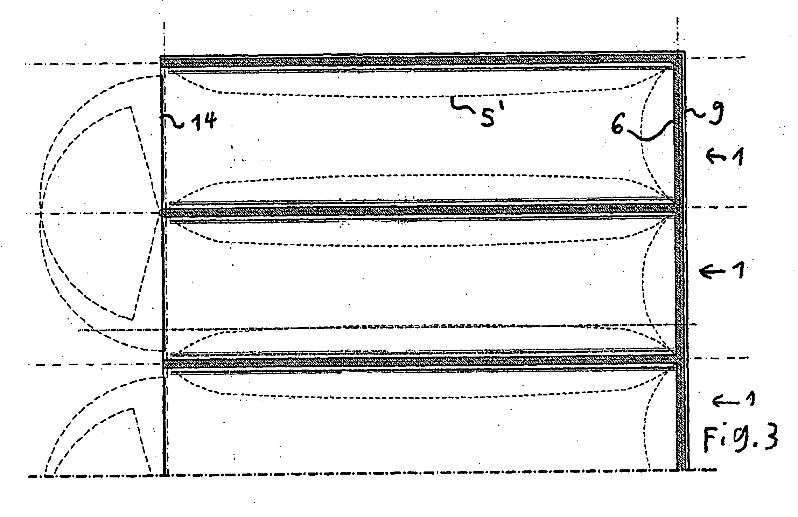

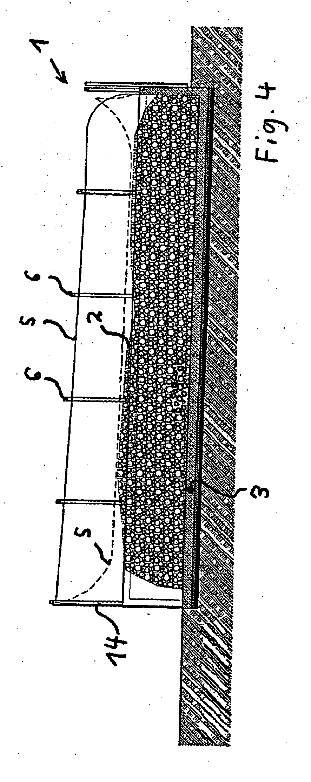

Die erfindungsgemäßen Fermenter können ebenfalls mit oder ohne eine weitere wärmeisolierende Umhüllung 9 nebeneinander angeordnet werden, wie dies in Figur 3 gezeigt ist. Dabei werden vertikale Elemente der starren Tragstruktur 6 sowie die starren Wände 8 von benachbarten Fermentern 1 gemeinsam genutzt. Die Sohle 3 des Fermenters kann so ausgeführt sein, dass am Aufstellungsort nur eine minimale Vorbereitung des Bodens, beispielsweise durch Planieren, erfolgen muss, so dass die Sohle 3 dem Fermenter 1 als Fundament dient, wie sie in Figur 4 dargestellt ist. Alternativ können weitere Fundamente vorgesehen sein. Ebenso ist es möglich, Unterkellerungen 13 am Aufstellungsort der Fermenter vorzusehen, die beispielsweise als Speicher für flüssige Medien und/oder Biogas dienen können. Weiterhin ermöglichen die starren Tragstrukturen der Fermenter, dass Zusatzmodule, die beispielsweise containerartig ausgeführt werden können, vorzugsweise zur Aufnahme technischer Infrastruktur, beispielsweise Pumpen, Wärmetauscher, Mess- und Regeltechnik oder Verdichter dienen, die nicht nur neben, sondern auch in Aufstellungsfläche sparender Weise auf den Fermenter 1 aufgestellt werden können.

Weiterhin ist es möglich, dass in dem erfindungsgemäßen Fermenter 1 eine Vorrichtung 19 zur Abtragung und/oder Führung eines Biomasseförder- oder Transportsystems vorgesehen ist. Dabei kann es sich beispielsweise um ein Schienensystem handeln, welches ein Biomasseförder- oder Transportsystem, welches beispielsweise ein verfahrbares Förderband sein kann, in einer Bewegungsrichtung entlang des Fermenters führt. Dabei ist die Vorrichtung 19 zur Abtragung und/oder Führung eines Biomasseförder- oder Transportsystems vorzugsweise so ausgelegt, dass die Last des Biomasseförder- oder Transportsystems über die Vorrichtung 19 abgetragen werden kann, so dass keine weitere Abstützung des Biomasseförder- oder Transportsystems auf dem Fermenterboden mehr notwendig ist, was sich positiv im Hinblick auf die Leichtbaukonstruktion der Sohle auswirkt. Die Last wird dabei vorzugsweise über starre Bereiche der Seitenwände bzw. die starre Tragstruktur 6 des Fermenters 1 abgetragen.

## Patentansprüche

1. Fermenter (1) zur Vergärung von Biomasse (2), welcher gasdicht ausgebildet ist, mit einer Sohle und einer flüssigkeitsdurchlässigen Stützvorrichtung (4) zur Abstützung und/oder Aufnahme der in den Fermenter (1) eingebrachten Biomasse (2), wobei der Fermenter (1) durch das Zusammenwirken wenigstens der Sohle mit einer die Fermenterkonstruktion nicht tragende Hülle (5) gasdicht verschlossen ist, welche so angebracht ist, dass die in dem Fermenter (1) eingebrachte Biomasse (2) sich lediglich auf der Sohle (3) und der Stützvorrichtung (4) abstützt, wobei der Fermenter (1) ein Drainagesystem (15) aufweist
**dadurch gekennzeichnet,**
**dass** die Sohle ein Belüftungssystem (7) sowie das Drainagesystem (15) aufweist, wobei das Drainagesystem (15) auf der von der eingebrachten Biomasse (2) weg weisenden Seite der Stützvorrichtung (4) angeordnet ist.

2. Fermenter (1) nach Anspruch 1,
**dadurch gekennzeichnet,**
**dass** eine starre Tragstruktur (6), die vorzugsweise nach Art eines Gitters ausgebildet ist, zur Abstützung und/oder Aufhängung der die Fermenterkonstruktion nicht tragenden Hülle (5) vorgesehen ist.

3. Fermenter (1) nach Anspruch 2,
**dadurch gekennzeichnet,**
**dass** eine Vorrichtung (19) zur Abtragung und/oder Führung eines Biomasseförder- oder Transportsystems vorgesehen ist.

4. Fermenter (1) nach einem der vorigen Ansprüche,
**dadurch gekennzeichnet,**
**dass** die die Fermenterkonstruktion nicht tragenden Hülle (5) nach Art eines Sackes ausgebildet ist.

5. Fermenter (1) nach einem der vorigen Ansprüche,
**dadurch gekennzeichnet,**
**dass** die die Fermenterkonstruktion nicht tragenden Hülle (5) eine Kunatstofffolie als wesentlichen Bestandteil aufweist.

6. Fermenter (1) nach einem der Ansprüche 2 bis 5,
**dadurch gekennzeichnet,**
**dass** die die Fermenterkonstruktion nicht tragenden Hülle (5) innerhalb der starren Tragstruktur (6) angeordnet ist.

7. Fermenter (1) nach einem der Ansprüche 2 bis 6,
**dadurch gekennzeichnet,**
**dass** die die Fermenterkonstruktion nicht tragenden Hülle (5) flexibel und an der starren Tragstruktur (6), vorzugsweise über eine höhenverstellbare Beriesclungseinrichtung (17), aufgehängt ist.

8. Fermenter (1) nach einem der vorigen Ansprüche,
**dadurch gekennzeichnet,**
**dass** die Sohle (3) eine Bodenplatte aus Beton oder verstärktem Beton, insbesondere Stahlbeton, aufweist.

9. Fermenter (1) nach einem der vorigen Ansprüche,
**dadurch gekennzeichnet,**
**dass** die die Fermenterkonstruktion nicht tragenden Hülle (5) in einem unteren Bereich zumindest einer Seitenwand als starre Wand (8) ausgeführt ist.

10. Fermenter (1) nach einem der vorigen Ansprüche,
**dadurch gekennzeichnet,**
**dass** eine Mehrzahl Fermenter (1) in einer weiteren Umhüllung (9) angeordnet sind, wobei die weitere Umhüllung (9) einen niedrigeren Wärmedurchgangskoeffizienten aufweist als die die Fermenterkonstruktion nicht tragenden Hülle (5).

11. Fermenter (1) nach einem der vorigen Ansprüche,
**dadurch gekennzeichnet,**
**dass** die Sohle (3) Durchlässe für ein flüssiges Medium aufweist.

12. Fermenter (1) nach einem der vorigen Ansprüche,
**dadurch gekennzeichnet,**
**dass** eine Leitung (11), vorzugsweise eine Steigleitung, für das flüssige Medium vorgesehen ist, die sich vorzugsweise in Höhenrichtung des Fermenters (1) mindestens vom Bereich der Sohle (3) bis zur für die eingebrachte Biomasse (2) vorgesehenen Haufwerkshöhe erstreckt.

## Claims

1. Fermenter (1) for fermenting biomass (2) which is made gas-tight, with a base plate and a fluid-permeable support device (4) for supporting and/or receiving the biomass (2) introduced into the fermenter (1), wherein the fermenter (1) is closed gas-tight by the interaction of at least the base plate with a cover (5) which does not support the fermenter structure and which is attached so that the biomass (2) introduced into the fermenter (1) is supported only on the base plate (3) and the support device (4) wherein the fermenter (1) has a drainage system (15),
**characterised in that**
the base plate has a ventilation system (7) as well as the drainage system (15) wherein the drainage system (15) is mounted on the side of the support device (4) which points away from the introduced biomass (2).

2. Fermenter (1) according to claim 2,
**characterised in that**
a rigid support structure (6) which is preferably designed in the manner of a mesh grid, is provided for supporting and/or suspending the cover (5) which does not support the fermenter structure.

3. Fermenter (1) according to claim 2,
**characterised in that**
a device (19) is provided for supporting and/or guiding a biomass conveyor or transport system.

4. Fermenter (1) according to one of the preceding claims
**characterised in that**
the cover (5) not supporting the fermenter structure is designed in the manner of a bag.

5. Fermenter (1) according to one of the preceding claims
**characterised in that**
the cover (5) not supporting the fermenter structure has a plastics foil as the essential constituent part.

6. Fermenter (1) according to one of claims 2 to 5
**characterised in that**
the cover (5) not supporting the fermenter structure is mounted inside the rigid support structure (6).

7. Fermenter (1) according to one of claims 2 to 6
**characterised in that**
the cover (5) not supporting the fermenter structure is suspended flexibly from the rigid support structure (6), preferably via a vertically adjustable sprinkling system (17).

8. Fermenter (1) according to one of the preceding claims
**characterised in that**
the base plate (3) comprises a foundation plate of concrete or reinforced concrete, more particularly steel-reinforced concrete.

9. Fermenter (1) according to one of the preceding claims
**characterised in that**
the cover (5) not supporting the fermenter structure is designed in a lower area of at least one side wall as a rigid wall (8).

10. Fermenter (1) according to one of the preceding claims
**characterised in that**
a number of fermenters (1) are arranged in a further enclosure (9) wherein the further enclosure (9) has a lower heat transfer coefficient than the cover (5) not supporting the fermenter structure.

11. Fermenter (1) according to one of the preceding claims
**characterised in that**
the base plate (3) has ports for a fluid medium.

12. Fermenter (1) according to one of the preceding claims
**characterised in that**
a pipe (11), preferably a riser pipe, is provided for the fluid medium which extends preferably in the vertical direction of the fermenter (1) at least from the area of the base plate (3) up to the top level of the pile of biomass which is introduced.

## Revendications

1. Fermenteur (1) destiné a la fermentation de biomasse (2), qui est de construction étanche aux gaz, avec une sole et un dispositif de support (4) perméable aux liquides, qui est destiné à soutenir et / ou à recevoir la biomasse (2) introduite dans le fermenteur (1), sachant que ledit fermenteur (1) est fermé de manière étanche aux gaz par la coopération d'au moins la sole (3) et d'une enveloppe (5), qui ne supporte pas la structure du fermenteur, mais qui est agencée de telle sorte que la biomasse (2) introduite dans le fermenteur (1) soit supportée seulement par la sole (3) et par le dispositif de support (4), sachant que le fermenteur (1) est doté d'un système de drainage (15),
**caractérisé en ce que**
la sole présente un système d'aération (7) ainsi que le système de drainage (15), sachant que ledit système de drainage (15) est disposé sur le côte du dispositif de support (4), qui est orienté à l'opposé de la biomasse (2) amenée.

2. Fermenteur (1) selon la revendication 1,
**caractérisé en ce que**,
pour soutenir et/ou suspendre l'enveloppe (5) ne supportant pas la structure du fermenteur, est prévue une structure de support rigide (6), qui, de préférence, est réalisée à la manière d'une grille.

3. Fermenteur (1) selon la revendication 2,
**caractérisé en ce**
**qu'**un dispositif (19) est prévu pour le déchargement et /ou le guidage d'un système d'alimentation ou de transport de biomasse.

4. Fermenteur (1) selon l'une des revendications précédentes,
**caractérisé en ce**
**que** l'enveloppe (5) ne supportant pas la structure du fermenteur est réalisée à la manière d'un sac.

5. Fermenteur (1) selon l'une des revendications précédentes,
**caractérisé en ce**
**que** l'enveloppe (5) ne supportant pas la structure du fermenteur comprend une feuille de matière synthétique en tant que composant essentiel.

6. Fermenteur (1) selon l'une des revendications 2 à 5,
**caractérisé en ce**
**que** l'enveloppe (5) ne supportant pas la structure du fermenteur est disposée à l'intérieur de la structure de support rigide (6)

7. Fermenteur (1) selon l'une des revendications 2 à 6,
**caractérisé en ce**
**que** l'enveloppe (5) ne supportant pas la structure du fermenteur est flexible et est suspendue à la structure de support rigide (6) par l'intermédiaire d'une installation d'irrigation par ruissellement (17) réglable en hauteur.

8. Fermenteur (1) selon l'une des revendications précédentes,
**caractérisé en ce**
**que** la sole (3) est dotée d'une plaque de sol en béton ou en béton armé, de préférence en béton à armature d'acier.

9. Fermenteur (1) selon l'une des revendications précédentes,
**caractérisé en ce**
**que** l'enveloppe (5) ne supportant pas la structure du fermenteur est réalisée, dans une zone inférieure d'au moins une paroi latérale, en tant que paroi rigide (8).

10. Fermenteur (1) selon l'une des revendications précédentes,
**caractérisé en ce**
**que** plusieurs fermenteurs (1) sont disposés dans une autre enveloppe (9), sachant que l'autre enveloppe (9) présente un coefficient de passage de chaleur plus faible que l'enveloppe (5) ne supportant pas la structure du fermenteur.

11. Fermenteur (1) selon l'une des revendications précédentes,
**caractérisé en ce**
**que** la sole (3) présente des passages pour un agent liquide.

12. Fermenteur (1) selon l'une des revendications précédentes,
**caractérisé en ce**
**qu'**est prévue une conduite (11), de préférence une conduite montante, pour l'agent liquide, laquelle s'étend de préférence dans le sens de la hauteur du fermenteur (1), au moins dans la région de la sole (3), jusqu'à la hauteur de déversement prévue pour la biomasse (2) amenée.
